(19) Europäisches Patentamt
European Patent Office
Office européen des brevets

(11) EP 1 976 842 B1

(12) **EUROPEAN PATENT SPECIFICATION**

(45) Date of publication and mention of the grant of the patent:
**10.03.2010 Bulletin 2010/10**

(21) Application number: **07702835.5**

(22) Date of filing: **17.01.2007**

(51) Int Cl.:
***C07D 295/20*** *(2006.01)* ***A61P 25/28*** *(2006.01)*
***A61P 29/00*** *(2006.01)*

(86) International application number:
**PCT/EP2007/000381**

(87) International publication number:
**WO 2007/082731 (26.07.2007 Gazette 2007/30)**

(54) **MODULATORS OF ALPHA7 NICOTINIC ACETYLCHOLINE RECEPTORS AND THERAPEUTIC USES THEREOF**

MODULATOREN VON ALPHA7-NIKOTIN-ACETYLCHOLIN-REZEPTOREN UND THERAPEUTISCHE ANWENDUNGEN DAVON

MODULATEURS DES RECEPTEURS NICOTINIQUES ALPHA7 DE L'ACETYLCHOLINE ET LEURS UTILISATIONS THERAPEUTIQUES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **18.01.2006 US 743142 P**

(43) Date of publication of application:
**08.10.2008 Bulletin 2008/41**

(73) Proprietor: **Siena Biotech S.p.A.**
**53100 Siena (IT)**

(72) Inventors:
- **BOTHMANN, Hendrick**
  **I-53100 Siena (IT)**
- **RONCARATI, Renza**
  **I-53100 Siena (IT)**
- **MICCO, Iolanda**
  **I-53100 Siena (IT)**
- **NENCINI, Arianna**
  **I-53100 Siena (IT)**
- **GHIRON, Chiara**
  **I-53100 Siena (IT)**

(74) Representative: **Banfi, Paolo**
**Bianchetti Bracco Minoja S.r.l.**
**Via Plinio, 63**
**20129 Milano (IT)**

(56) References cited:
**WO-A-98/05292** **WO-A1-01/81310**

- **SHIMOGAWA H ET AL: "A WRENCH-SHAPED SYNTHETIC MOLECULE THAT MODULATES A TRANSCRIPTION FACTOR-COACTIVATOR INTERACTION" JOURNAL OF THE AMERICAN CHEMICAL SOCIETY, AMERICAN CHEMICAL SOCIETY, WASHINGTON, DC, US, vol. 126, no. 11, 24 February 2004 (2004-02-24), pages 3461-3471, XP002389913 ISSN: 0002-7863**

EP 1 976 842 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The present invention relates to compounds with α7 nicotinic acetylcholine receptor (α7 nAChR) agonistic activity, processes for their preparation, pharmaceutical compositions containing the same and the use thereof for the treatment of neurological and psychiatric diseases.

## Background of the invention

A number of recent observations point to a potential neuroprotective effect of nicotine in a variety of neurodegeneration models in animals and in cultured cells, involving excitotoxic insults (1-5), trophic deprivation (6), ischemia (7), trauma (8), Aß-mediated neuronal death (9-11) and protein-aggregation mediated neuronal degeneration (9;12). In many instances where nicotine displays a neuroprotective effect, a direct involvement of receptors comprising the α7 subtype has been invoked (7;11-15) suggesting that activation of α7 subtype-containing nicotinic acetylcholine receptors may be instrumental in mediating the neuroprotective effects of nicotine. The available data suggest that the α7 nicotinic acetylcholine receptor represents a valid molecular target for the development of agonists/positive modulators active as neuroprotective molecules. Indeed, α7 nicotinic receptor agonists have already been identified and evaluated as possible leads for the development of neuroprotective drugs (16-20). Involvement of α7 nicotinic acetylcholine receptor in inflammatory processes has also recently been described (21). Thus, the development of novel modulators of this receptor should lead to novel treatments of neurological, psychiatric and inflammatory diseases.

## Summary of the invention

The invention provides compounds acting as full or partial agonists at the α7 nicotinic acetylcholine receptor (α7 nAChR), pharmaceutical compositions containing the same compounds and the use thereof for the treatment of diseases that may benefit from the activation of the alpha 7 nicotinic acetylcholine receptor such as neurological and psychiatric disorders, in particular Alzheimer's disease and schizophrenia.

## Known prior art

Different heterocyclic compounds carrying a basic nitrogen and exhibiting muscarinic acetylcholine receptor affinity or claimed for use in Alzheimer disease were found to be described, e.g. compounds with activity on muscarinic receptors (US652852; WO2001005763; WO9950247); substituted 1-piperidin-4-yl-4-pyrrolidin-3-yl piperazine derivatives (WO2004056799); preparation of substituted 4-(4-piperidin-4-yl-piperazin-1-yl)-azepane derivatives (WO2004056805); novel non-imidazole compounds (WO02072570); substance P antagonists (WO0130348).

1-(1,2-disubstituted piperidinyl)-4-substituted piperazines and substituted 1,4-di-piperidin-4-yl-piperazine derivatives are reported in WO200411045 and WO2004110415, respectively.

## Description of the invention

In a first aspect, the invention provides a compound of formula I

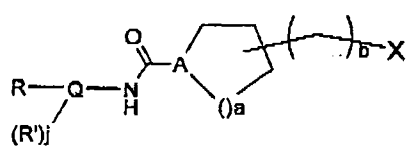

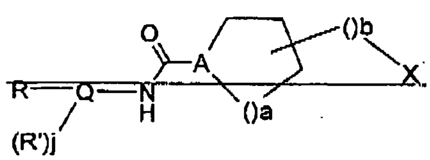

(I)

wherein:

A is N;
a is an integer from 1 to 3;
b is 0, 1, or 2;
X is a group of formula:

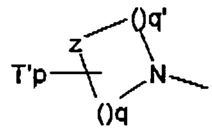

wherein:

Z is $CH_2$, N, O;
p is 0 or 1;
T' represent, independently from one another when p is greater than 1, linear, branched or cyclic ($C_1$-$C_6$) alkyl, trihaloalkyl, hydroxyalkyl, alkoxy, alkylcarbonyl, alkoxycarbonyl, alkylcarbonylamino; linear, branched or cyclic ($C_1$-$C_6$) alkoxy-($C_1$-$C_6$) alkyl; linear, branched or cyclic ($C_1$-$C_6$) alkylaminocarbonyl; carbamoyl; or, when p is 2 or 3, two T' substituents form a 5- to 8-membered ring with spiro or fused junction;
q and q' are, independently from one another, integers from 1 to 4;
Q is a 5 to 10-membered aromatic or heteroaromatic ring;
R represents a 5 to 10-membered aromatic or heteroaromatic ring optionally substituted with one or more groups selected from: halogen; hydroxy; mercapto; cyano; nitro; amino; linear, branched or cyclic ($C_1$-$C_6$) alkyl, trihaloalkyl, alkoxy or alkylcarbonyl,; linear, branched, or cyclic ($C_1$-$C_6$) alkylcarbonylamino; mono- or di, linear, branched, or cyclic ($C_1$-$C_6$) alkylamino or alkylaminocarbonyl; carbamoyl; linear, branched or cyclic ($C_1$-$C_6$) alkoxy-($C_1$-$C_6$) alkyl;
j is 0, 1 or 2;
R' represent, independently from one another when j = 2, halogen; hydroxy; trihalomethyl; trihalomethoxy; linear, branched or cyclic ($C_1$-$C_6$) alkyl, trihaloalkyl, alkoxy, hydroxyalkyl.

Particularly preferred are those compounds of formula I wherein:

A is N;
a is an integer from 1 to 2;
b is 0, 1 or 2;
X is a group of formula:

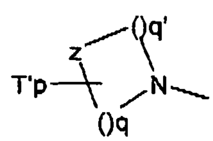

wherein:

Z is $CH_2$;
p is 0;
q and q' are, independently from one another, integers from 1 to 3;
Q is a 5 to 10-membered aromatic ring;
R represents a 5 to 10-membered aromatic or heteroaromatic ring optionally substituted with one or more groups selected from: halogen; linear, branched or cyclic ($C_1$-$C_3$) alkyl, alkoxy; linear, branched, or cyclic ($C_1$-$C_3$) alkylcarbonylamino; mono- or di, linear, branched, or cyclic ($C_1$-$C_3$) alkylaminocarbonyl; carbamoyl;
j is 0.

Yet more preferred are those compounds wherein both Q and R are phenyl.
The compounds of Formula I can be prepared through a number of synthetic routes amongst which the ones illustrated in Schemes 1, 2, and 3 below.

a) Scheme 1:

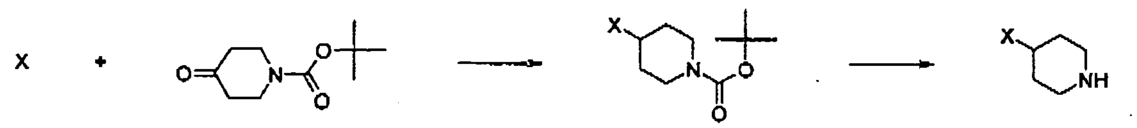

1 2 3 4

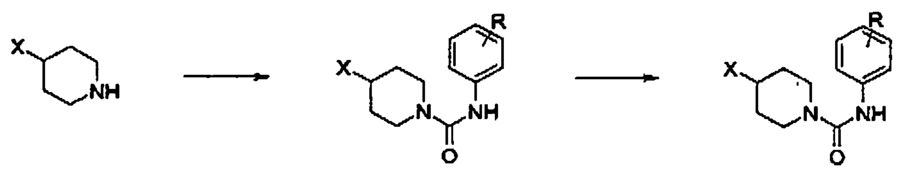

4 Iα Iβ

According to Scheme 1, hereby exemplified by a = 2 and A = N, an amine 1 is reacted under reductive alkylation conditions, such as for example treatment with sodium triacetoxyborohydride, sodium cyanoborohydride or sodium borohydride, in the presence of a catalytic amount of acid, such as for example acetic acid or formic acid, in an organic solvent such as for example dichloromethane or tetrahydrofuran, with a cyclic ketone 2 containing a protected amine functionality, hereby exemplified by tert-butoxycarbonyl. Other suitable protecting groups may be represented by benzyloxycarbonyl, fluorenylmethoxycarbonyl, and any other amine protecting group as described in Greene, T. and Wuts, P. G. M. Protective Groups in Organic Synthesis, John Wiley and Sons, 1999. The thus obtained amine 3 is further modified by removal of the protecting group, for example in the case of the *tert*-butoxycarbonyl group by treatment with trifluoroacetic acid in dichloromethane or with hydrochloric acid in methanol or with any other suitable method as described in Ref. 1, to obtain amine 4. Amine 4 is then reacted with an isocyanate, hereby exemplified by a phenylisocyanate, in a suitable solvent such as for example dichloromethane, tetrahydrofuran, dimethylformamide or mixtures thereof, to yield the ureas Iα. In the case of R being a halogen or a boronic acid ester, Iα can be further processed - for example via a cross-coupling reaction, for example under the conditions described as Suzuki coupling conditions (Suzuki, A. Pure and Appl. Chem. 1994 66 213-222), with a boronic acid or an aryl or heteroaryl halide - to yield compounds Iβ.

b) Scheme 2:

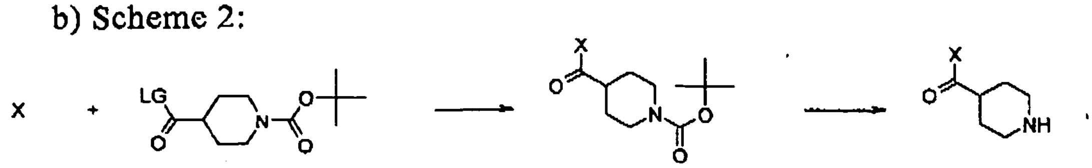

1 5 6 7

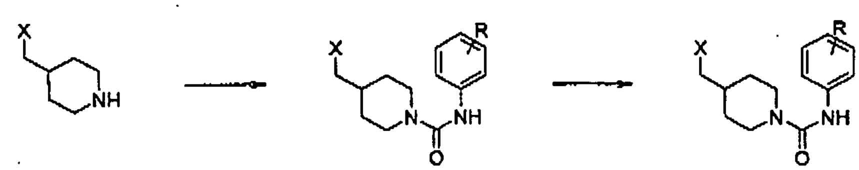

8 Iα Iβ

According to Scheme 2, hereby exemplified by a = 2, b = 1, and A = N, an amine 1 is reacted with an activated acid 5 (LG = leaving group) containing a protected amine functionality, hereby exemplified by *tert*-butoxycarbonyl, to afford an amide 6 in a solvent such as for example dichloromethane, tetrahydrofuran, dimethylformamide or mixtures thereof. Suitable activation for acids may be represented by acid chloride, an acyl imidazolide as obtained by treatment of an acid with a stoichiometric amount of carbonyldiimidazole, an activated ester such as for example a benzotriazolyl ester

or a pentafluorophenyl ester, a mixed anhydride such as for example the one obtained by reaction of an acid with a isobutyl chloroformate in the presence of a tertiary amine. The thus obtained amide 6 is further modified by removal of the protecting group, for example in the case of the *tert*-butoxycarbonyl group by treatment with trifluoroacetic acid in dichloromethane or with hydrochloric acid in methanol or any other suitable method as mentioned above, to obtain amine 7. Amine 7 is then reacted with a reducing agent, such as lithium aluminium hydride or borane in a suitable solvent such as for example tetrahydrofuran to afford amine 8, which is further reacted with an isocyanate, hereby exemplified by a phenylisocyanate, in a suitable solvent such as for example dichloromethane, tetrahydrofuran dimethylformamide or mixtures thereof, to yield the ureas Iα. In the case of R being a halogen or a boronic acid ester, Iα can be further processed - for example via a cross-coupling reaction, for example under the Suzuki coupling conditions, with a boronic acid or an aryl or heteroaryl halide - to (yield compounds Iβ.

c ) Scheme 3

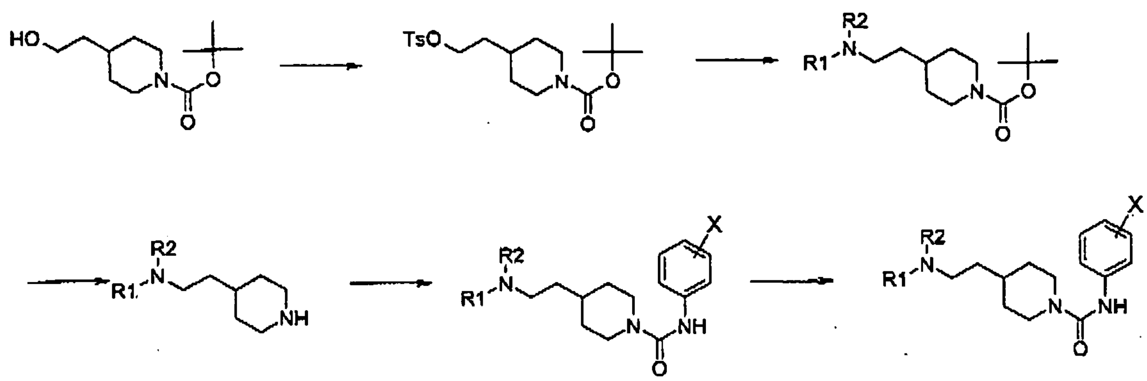

Iα

Iβ

According to Scheme 34, the hydroxy group of a suitably protected hydroxyalkylcyclamine, hereby exemplified, but not limited to, by 4-(2-hydroxyethyl)piperidine *N*-protected as its *tert*-butylcarbamate derivative, is activated by the transformation into a leaving group, hereby exemplified by, but not limited to, a *para*-toluenesulphonyl group and subsequently displaced in the reaction with a primary or secondary amine. Following removal of the cyclamine protecting group, this is then reacted with an aryl or heteroaryl isocyanate in a suitable solvent, such as for example dichloromethane, or tetrahydrofurane to furnish product Iα. In the case of X being a halogen or a boronic acid ester, Iα can be further processed - for example via a cross-coupling reaction, for example under the Suzuki coupling conditions, with a boronic acid or an aryl or heteroaryl halide - to yield compounds Iβ.

The compounds of formula I, their optical isomers or diastereomers can be purified or separated according to well-known procedures, including but not limited to chromatography with a chiral matrix and fractional crystallisation.

The pharmacological activity of a representative group of compounds of formula I was demonstrated in an *in vitro* assay utilising cells stably transfected with the alpha 7 nicotinic acetylcholine receptor and cells expressing the alpha 1 and alpha 3 nicotinic acetylcholine receptors and 5HT3 receptor as controls for selectivity. According to a further aspect, the invention is therefore directed to a method of treating neurological and psychiatric disorders, which comprises administering to a subject, preferably a human subject in need thereof, an effective amount of a compound of formula I. Neurological and psychiatric disorders that may benefit from the treatment with the invention compounds include but are not limited to senile dementia, attention deficit disorders, Alzheimer's disease and schizophrenia. In general, the compounds of formula I can be used for treating any disease condition, disorder or dysfunction that may benefit from the activation of the alpha 7 nicotinic acetylcholine receptor, including but not limited to Parkinson's disease, Huntington's chorea, amyotrophic lateral sclerosis, multiple sclerosis, epilepsy, memory or learning deficit, panic disorders, cognitive disorders, depression, sepsis and arthritis.

The dosage of the compounds for use in therapy may vary depending upon, for example, the administration route, the nature and severity of the disease. In general, an acceptable pharmacological effect in humans may be obtained with daily dosages ranging from 0.01 to 200 mg/kg.

In yet a further aspect, the invention refers to a pharmaceutical composition containing one or more compounds of formula I, in association with pharmaceutically acceptable carriers and excipients. The pharmaceutical compositions can be in the form of solid, semi-solid or liquid preparations, preferably in form of solutions, suspensions, powders, granules, tablets, capsules, syrups, suppositories, aerosols or controlled delivery systems. The compositions can be

administered by a variety of routes, including oral, transdermal, subcutaneous, intravenous, intramuscular, rectal and intranasal, and are preferably formulated in unit dosage form, each dosage containing from about 1 to about 1000 mg, preferably from 1 to 600 mg of the active ingredient. The compounds of the invention can be in the form of free bases or as acid addition salts, preferably salts with pharmaceutically acceptable acids. The invention also includes separated isomers and diastereomers of compounds I, or mixtures thereof (e.g. racemic mixtures). The principles and methods for the preparation of pharmaceutical compositions are described for example in Remington's Pharmaceutical Science, Mack Publishing Company, Easton (PA).

**Experimental Procedures - Synthesis of compounds**

*General*

Unless otherwise specified all nuclear magnetic resonance spectra were recorded using a Varian Mercury Plus 400 Mhz spectrometer equipped with a PFG ATB Broadband probe.

HPLC-MS analyses were performed with a Waters 2795 separation module equipped with a Waters Micromass ZQ (ES ionisation) and Waters PDA 2996, using a Waters XTerra MS C18 3.5 μm 2.1x50 mm column.

Preparative HLPC was run using a Waters 2767 system with a binary Gradient Module Waters 2525 pump and coupled to a Waters Micromass ZQ (ES) or Waters 2487 DAD, using a Supelco Discovery HS C18 5.0 μm 10x21.2 mm column.

Gradients were run using 0.1% formic acid/water and 0.1% formic acid/acetonitrile with gradient 5/95 to 95/5 in the run time indicated.

All column chromatography was performed following the method of Still, C.; J. Org Chem 43, 2923 (1978). All TLC analyses were performed on silica gel (Merck 60 F254) and spots revealed by UV visualisation at 254 nm and KmnO4 or ninhydrin stain.

When specified for array synthesis, heating was performed on a Buchi Syncore® system.

All microwave reactions were performed in a CEM Discover oven.

*Abbreviations used throughout the Experimental Procedures*

| | |
|---|---|
| DCM | dichloromethane |
| DCE | 1,2-dichloroethane |
| DMEA | N,N-dimethylethylamine |
| DMF | N,N-dimethylformamide |
| DMSO, dmso | dimethylsulphoxide |
| SCX | strong cation exchanger |
| TEA | triethylamine |
| TFA | trifluoroacetic acid |
| THF | tetrahydrofuran |
| TLC | thin layer chromatography |
| LC-MS | Liquid chromatography - mass spectrometry |
| HPLC | High performance liquid chromatography |

*General procedure for the reductive alkylation of N-Boc-piperidone*

The secondary amine (1.0 eq) was dissolved in DCM and N-boc-piperidone (1.0 eq) was added. The reaction was stirred for 1 hour and then $NaBH(OAc)_3$ was added and reaction stirred for other 18 hours.

The mixture was then extracted into a HCl solution at pH2 and non-basic impurities removed by washing with DCM. The aqueous phase was then brought to pH 12 with sodium hydroxide and extracted with DCM. The organic phase was concentrated under reduced pressure affording the reductive alkylation product pure enough for the following step.

*General procedure for the removal of Boc protecting group*

The product obtained from the above reductive alkylation step (1 eq) was dissolved in DCM and an excess of TFA (80 eq) was slowly added.

The reaction was stirred for 1h and then concentrated under reduced pressure. The mixture was neutralised with NaOH 10% and extracted with DCM, affording a piperidine product pure enough for the following step.

*General procedure for 4-(alkyl)aminomethylpiperidine synthesis*

To a suspension of N-Boc-isonipecotic acid (1 eq) and TBTU (1 eq) in $CH_3CN$, the appropriate amine (2 eq) was added. The resulting solution was stirred at 85°C for 6 hours.

The reaction mixture was concentrated under reduced pressure and then dissolved in DCM and washed twice with sat. aqueous $Na_2CO_3$ solution.

Solvent removal gave the 4-(alkyl)carbamoyl-piperidine-1-carboxylic acid tert-butyl ester product usually pure enough for the next step.

The amide thus obtained was dissolved in 6N HCl solution at 0°C. After 10 minutes TLC analysis generally showed disappearance of starting material, and the mixture was basified to pH 12 with NaOH (pellets) and extracted with AcOEt.

The organic phase concentrated under reduced pressure gave the piperidine-4-carboxylic acid amide product that was used without further purification for the next step.

The piperidine-amide was added (at 0°C) to a suspension of $LiAlH_4$ in anhydrous THF. After stirring for 30 minutes the reaction was heated at reflux for 1 hour and when TLC analysis generally showed complete conversion of the starting amide. The reaction was cooled to 0°C and $LiAlH_4$ was quenched with $H_2O$ and NaOH (10% aqueous solution). The inorganic salts were filtered and the solution was concentrated under reduced pressure, yielding the piperidin-4-yl methylamine product usually pure enough for the next step.

*General procedure for 4-(2-(N-alkylamino)ethylpiperidine synthesis*

*a) 4-[2-(Toluene-4-sulfonyloxy)-ethyl]-piperidine-1-carboxylic acid tert-butyl ester*

To a solution of 4-(2-Hydroxy-ethyl)-piperidine-1-carboxylic acid tert-butyl ester in DCM (0.65 mmol/mL) p-toluensulphonyl chloride (1.5 eq) and dimethylaminopyridine (1 eq) were added. The reaction was left at rt for 18h and then TLC showed complete conversion of the starting material.

The mixture was washed with NaOH 2N and then with HCl 2N, the organic phase was dried over $Na_2SO_4$ and then concentrated under reduced pressure. The oil obtained was purified with $SiO_2$ column, eluting with DCM, giving the pure product in quantitative yield.

1H-NMR (400MHz, CDCl3): 1.01-1.15 (m,2H), 1.44 (s, 9H), 1.47-1.57 (m, 2H), 1.62-1.72 (m, 3H), 2.44 (s, 3H), 2.54-2.65 (m, 2H), 3.95-4.1 (m, 4H), 7.36 (d, 2H, J=7.3), 7.78 (d, 2H, J=7.3).

*b) General procedure for 4-(2-(N-Alkylamino)-ethyl)-piperidine-1-carboxylic acid tert-butyl ester synthesis*

To the chosen alkylamine (2 eq) was added a solution of 4-[2-(toluene-4-sulfonyloxy)-ethyl]-piperidine-1-carboxylic acid tert-butyl ester in $CH_3CN$ (0.65 mmol/mL, 1eq) and the reaction was heated at 80°C for about 6 hours. Upon complete conversion (as monitored by thin layer chromatography) the mixture was cooled down to room temperature and washed with saturated NaCl water solution. The organic phase was dried over $Na_2SO_4$ and then concentrated under reduced pressure. The oil obtained was purified by $SiO_2$ column eluting with gradient starting from 100% DCM to DCM-NH3(2N MeOH solution) 9:1.

*c) General procedure for 4-(2-(N-alkylamino)-ethyl)-piperidine synthesis*

To a solution of HCl 6N (30 eq) was added the 4-(2-(N-alkylamino)-ethyl)-piperidine-1-carboxylic acid tert-butyl ester and the reaction was stirred at room temperature for 10 minutes. The mixture was basified to pH 12 and the product was extracted with DCM. The organic phase was dried over $Na_2SO_4$ and then concentrated under reduced pressure affording the pure product.

*General procedure for urea synthesis*

To a cooled solution of amine (1 eq) in dichloromethane an equimolar amount of an aryl or heteroaryl isocyanate was added. In the case of the amine being in the form of a hydrochloride or bis-hydrochloride salt, equimolar amounts of TEA were added to free-base the amine.

The mixture was left stirring at 0°C for 1-4 hours. The *p*-bromophenyl ureas generally precipitated out of solution as white solids, were recovered by filtration and if necessary purified further by washing with $Et_2O$ or by flash chromatography. The m-bromophenylureas were isolated by solvent removal under reduced pressure and purified by crystallisation from an mixture of AcOEt: $Et_2O$.

*General procedure for cross-coupling reaction - thermal conditions*

To a degassed solution of aryl or heteroaryl bromide prepared following the general procedure for urea synthesis described above (1 eq), the appropriate boronic acid (1.3 eq) was added dissolved in 40 volumes (Wt/vol) of acetonitrile/0.4N aqueous $Na_2CO_3$ (1/1), $Pd[(PPh_3)]_4$ (10% mol). The solution was refluxed overnight under nitrogen either in a round-bottom flask or in a glass test tube in a Buchi SynCore® apparatus.

The acetonitrile phase was separated and the desired products purified over a SCX or silica column. Fractions containing the desired product were combined and dried under reduced pressure.

*General procedure for cross-coupling reaction - microwave conditions - $Pd[(PPh_3)]_4$*

To a degassed solution of bromide prepared following the general procedure for urea synthesis (1 eq), the appropriate boronic acid (1 eq) and $Na_2CO_3$ (3 eq) in 20 volumes (Wt/vol) of acetonitrile/water (1/1), $Pd[(PPh_3)]_4$ (10% mol) were added.

The solution was irradiated with microwave using following parameters: power 200 watt; ramp time 1 min; hold time 20 min; temperature 90°C; pressure 200 psi.

The acetonitrile phase was separated, the solvent was removed under reduced pressure and the crude material purified using SCX column (eluting with a gradient of DCM/MeOH, MeOH, $NH_3$/MeOH). The fractions containing the desired product were combined and dried under reduced pressure.

*General procedure for cross-coupling reaction - microwave conditions - Tri-o-tolylphosphine*

To a degassed solution of aryl/heteroaryl bromide prepared following the general procedure(1 eq), in DME/$H_2O$ (1.8/0.3), the appropriate boronic acid.
1.5 eq) $Na_2CO_3$ (2 eq), $Pd(OAc)_2$ (10% mol) and tri-o-tolylphosphine(40% mol), were added. The solution was irradiated under microwave conditions for 20 minutes at power = 200W.

The organic phase was separated and the desired products purified using SCX column and/or prep-HPLC. Fractions containing the desired product were combined and dried under reduced pressure.

*Example 1*

*4-Azepan-1-yl-piperidine-1-carboxylic acid (2'-chloro-biphenyl-4-yl)-amide*

*a) 4-Azepan-1-yl-piperidine-1-carboxylic acid tert-butyl ester*

Azepine (0.99 g, 10 mmol) was dissolved in 20 ml of DCM and N-boc-piperidone (2.58 g, 13 mmol) was added. The reaction was stirred for 1 hour and then $NaBH(OAc)_3$ (3.16 g, 15 mmol) was added and the mixture stirred for further 18 hours.

The mixture was extracted with HCl solution at pH2 and then the aqueous phase was basified to pH 12 and extracted with DCM. The organic phase was concentrated under reduced pressure affording the title product (1.7 g, 60% yield).

C16H30N2O2 Mass (calculated) [282.43]; (found) $[M+H^+]$=283.

$^1$H-NMR (400 MHz, CDCl3) 1.29-1.52 (11H, m); 1.53-1.67 (8H, m); 1.67-1.81 (2H, m); 2.48-2.80 (7H, m); 3.98-4.28 (2H, m).

*b) 1-Piperidin-4-yl-azepane*

4-Azepan-1-yl-piperidine-1-carboxylic acid tert-butyl ester (1.7 g, 6.1 mmol) was dissolved in 10 ml of DCM and 10 ml of TFA was slowly added.

The reaction was stirred for 1 hour and then concentrated under reduced pressure. The mixture was neutralised with NaOH 10% and extracted with DCM, affording the title compound (800 mg, 72% yield).

C11H22N2 Mass (calculated) [182.31]; (found) $[M+H^+]$=183.

LcRt (5 min method)= 0.37.

$^1$H-NMR (400 MHz, CDCl3) 1.30-1.46 (2H, m), 1.49-1.67 (9H, m), 1.70-1.82 (2H, m), 2.44-2.61 (3H, m), 2.63-2.67(4H, m), 3.04-3.17 (2H, m).

*c) 4-Azepan-1-yl-piperidine-1-carboxylic acid (4-bromo-phenyl)-amide*

To a cooled solution of 1-piperidin-4-yl-azepane (0.80 g, 4.4 mmol) in dichloromethane (20 mL), 4-bromophenylisocyanate (0.88 g, 4.4 mmol) was added. The mixture was stirred at 0˚C until precipitation of a white solid was observed after 2 hours.

The white solid was filtered and washed with $Et_2O$ to afford 1.49 g of the title product (90% yield).

C18H26BrN3OMass (calculated) [380.33]; (found) $[M+H^+]$=380/382 (Br).

Lc Rt (5 min method)= 1.63, 92%.

NMR (400 MHz, DMSO): 1.19-1.39 (2H, m); 1.43-1.58 (8H, m); 1.61-1.63 (2H, m); 2.52-2.65 (4H, m); 2.65-2.82 (2H, m); 4.03-4.19 (2H, m); 7.36 (2H, d, J=8 Hz); 7.42 (2H, d, J=8 Hz), 8.57 (1H, s).

*d) 4-Azepan-1-yl-piperidine-1-carboxylic acid (2'-chloro-biphenyl-4-yl)-amide*

4-Azepan-1-yl-piperidine-1-carboxylic acid (4-bromo-phenyl)-amide was weighed (0.1 g, 0.26 mmol), placed in a glass test tube and dissolved in 4 mL of a degassed solution of acetonitrile/0.4N aqueous $Na_2CO_3$ (1/1). To this solution, 2-chlorophenyl boronic acid (0.066 g, 0.42 mmol) and $Pd[P(Ph)_3]_4$ (10% mol) were added. The mixture was heated at 80˚C and shaken in a Buchi SynCore® for 18 hours.

The solution was diluted with AcOEt and the organic phase was separated, and dried under reduced pressure; the crude was purified over a $SiO_2$ column (eluent: gradient from DCM to DCM/MeOH 9/1). The fractions containing the product were collected and dried under reduced pressure (14% yield).

C24H30ClN3O Mass (calculated) [411.98]; (found) $[M+H^+]$= 412.

Lc Rt: 2.89, 100%.

*Example 2*

*[1,4']Bipiperidinyl-1'-carboxylic acid (2'-chloro-biphenyl-4-yl)-amide*

[1,4']Bipiperidinyl-1'-carboxylic acid (4-bromo-phenyl)-amide was weighed (0.1 g, 0.28 mmol), placed in a glass test tube and dissolved with 4 mL of a previously degassed solution of acetonitrile/0.4N aqueous $Na_2CO_3$ (1/1). To this solution, 2-chlorophenyl boronic acid (0.066 g, 0.42 mmol) and $Pd[P(Ph)_3]_4$ (10% mol equivalents) were added. The mixture was heated at 80˚C and shaken in a Buchi SynCore® for 18 hours.

The solution was diluted with AcOEt and the organic phase was separated, and dried under reduced pressure; the crude was purified over a $SiO_2$ column (eluent: gradient from DCM to DCM/MeOH 9/1). The fractions containing the product were collected and dried under reduced pressure to give the title compound (13% yield).

C23H28ClN3OMass (calculated) [397.95]; (found) $[M+H^+]$= 398.

Lc Rt (10 min method): 2.83, 97%.

*Example 3*

*4-Pyrrolidin-1-yl-piperidine-1-carboxylic acid (3'-carbamoyl-biphenyl-4-yl)-amide*

4-Pyrrolidin-1-yl-piperidine-1-carboxylic acid (4-bromo-phenyl)-amide was weighed (0.1 g, 0.30 mmol), placed in a glass test tube and dissolved in 4 mL of a degassed solution of acetonitrile/0.4N aqueous $Na_2CO_3$ (1/1). To this solution, 3-benzamide-phenyl boronic acid (0.069 g, 0.42 mmol) and $Pd[P(Ph)_3]_4$ (10% mol) were added. The mixture was heated at 80˚C and shaken in a Buchi SynCore® for 18 hours.

The solution was diluted with AcOEt and the organic phase was separated, and dried under reduced pressure; the crude was purified over a $SiO_2$ column (eluent: gradient from DCM to DCM/MeOH 9/1). The fractions containing the product were collected and dried under reduced pressure (28% yield).

C23H28N4O2Mass (calculated) [392.51]; (found) $[M+H^+]$= 393.

Lc Rt (10 min method): 0.33-1.78 (double peak), >90%.

$^1$H-NMR (CD3OD):1.30-1.48 (2H, m), 1.65-1.79 (4H, m), 1.85-2.01 (1H, m), 2.49-2.66 (4H, m), 2.76-2.92 (2H, m), 4.06-4.21(2H, m), 7.36-7.47 (3H, m), 7.49-7.56 (2H, m), 7.68-7.75 (1H, m), 8.03 (1H, s).

*Example 4*

*4-Piperidin-1-ylmethyl-piperidine-1-carboxylic acid (2'-fluoro-biphenyl-4-yl)-amide*

*a) 4-(Piperidine-1-carbonyl)-piperidine-1-carboxylic acid tert-butyl ester*

To a suspension of N-Boc-isonipecotic acid (3.0 g, 13.1 mmol) and TBTU (4.2 g, 13.1 mmol) in 60 mL of $CH_3CN$, piperidine (1.67 g, 19.6 mmol) was added. The resulting solution was stirred at 85°C for 6 hours.

The reaction mixture was concentrated under reduced pressure and then dissolved in DCM and washed twice with saturated aqueous $Na_2CO_3$ solution.

Solvent removal gave 3.2 g of title compound that was used without further purification in the next step.

*b) Piperidin-4-yl-piperidin-1-yl-methanone*

3.2 g (10.8 mmol) of 4-(piperidine-1-carbonyl)-piperidine-1-carboxylic acid tert-butyl ester were dissolved in 25 mL of 6N HCl solution at 0°C.

After 10 minutes TLC showed complete conversion of the starting material, the mixture was cooled at 0°C, basified to pH 10 with NaOH (pellets) and extracted with AcOEt. The organic phase concentrated under reduced pressure gave 1.9 g of title compound (91% yield).

NMR (400 MHz, DMSO): 1.43-1.59 (4H, m), 1.60-1.75 (8H, m), 2.53-2.71 (3H, m), 3.06-3.20 (2H, m), 3.36-3.47 (2H, m), 3.50-3.61 (2H, m).

*c) 4-Piperidin-1-ylmethyl-piperidine*

1.9 g (9.8 mmol) of piperidin-4-yl-piperidin-1-yl-methanone were added (at 0°C) to a suspension of 0.74 g (17.7 mmol) of $LiAlH_4$ in anhydrous THF. After stirring for 30 minutes the reaction was heated at reflux for 1 hour when TLC analysis showed complete conversion of the starting amide.

The reaction was cooled to 0°C and $LiAlH_4$ was quenched with 0.7 ml of $H_2O$ and 2.8 ml of NaOH (10% aqueous solution). The inorganic salts were filtered and the solution was concentrated under reduced pressure, affording 1.2 g of the title compound, with NMR purity about 80%, that was used for the following step.

NMR (400 MHz, CDCl3): 0.99-1.23 (2H, m), 1.30-1.45 (2H, m), 1.46-1.67 (4H, m), 1.67-1.77 (2H, m), 2.15-2.37 (4H, m), 2.52-2.63 (1H, m), 3.02-3.11 (1H, m).

*d) 4-Piperidin-1-ylmethyl-piperidine-1-carboxylic acid (4-bromophenyl)-amide*

To a cooled solution of 4-piperidin-1-ylmethyl-piperidine (1.2 g, 80% purity, 6.3 mmol) in dichloromethane (20 mL) p-bromophenylisocyanate (1.24 g, 6.3 mmol) was added and the mixture stirred at 0°C until a white solid precipitated out of solution after 2 hours. The white solid was filtered off and washed with $Et_2O$ to give 1.2 g of pure title compound (50% yield).

Molecular formula: C18H26BrN3O.

Mass (calculated) [380.33]; (found) $[M+H^+]$=380-382.

Lc Rt (10 min method)= 2.11, 99%.

NMR (400 MHz, DMSO): 0.81-1.07 (2H, m), 1.29-1.39 (2H, m), 1.40-1.50 (4H, m), 1.57-1.73 (3H, m), 1.95-2.08 (2H,m), 2.15-2.35 (4H, m), 2.66-2.77 (2H, m), 4.00-4.10 (2H, m), 7.35 (2H, d, J=8.8 Hz), 7.41 (2H, d, J=8.8Hz), 8.54 (1H, s).

To a degassed solution of 4-Piperidin-1-ylmethyl-piperidine-1-carboxylic acid (4-bromo-phenyl)-amide (100 mg, 0.26 mmol) in DME/$H_2O$ (1.8 ml/0.3 ml) 2-fluorophenyl boronic acid (55 mg, 0.39 mmol), $Na_2CO_3$ (55 mg, 0.52 mmol), $Pd(OAc)_2$ (6 mg, 10% mol) and tri-o-tolylphosphine (34 mg, 20% mol), were added. The solution was irradiated under microwave conditions for 20 minutes with power 200W. The organic phase was then diluted with 1 mL of AcOEt, separated and loaded on a SCX column and eluted with 10 mL of MeOH to remove triphenylphosphinoxide and then with NH3 (2N MeOH solution) to recover the pure product (55 mg, 56% yield).

Molecular formula: C24H30FN3O.

Mass (calculated) [395]; (found) $[M+H^+]$=396.

Lc Rt (10 min method)= 2.73, 99%.

$^1$H-NMR (400MHz, d6-DMSO): 0.92-1.09 (m,2H), 1.28-1.40 (m, 2H), 1.41-1.56 (m, 4H), 1.61-1.79 (m, 3H), 2.01-2.11 (m, 2H), 2.12-2.37 (m, 4H), 2.69-2.84 (m,2H), 4.00-4.19 (m, 2H), 7.22-7.29 (m,2H), 7.31-7.36 (m,1H), 7.38-7.44 (m, 2H), 7.43-7.56 (m, 1H), 7.51-7.59 (m, 2H), 8.57 (s,1H).

*Example 5*

*4-(2-Piperidin-1-yl-ethyl)-piperidine-1-carboxylic acid (2'-fluoro-biphenyl-4-yl)-amide*

*a) 4-(2-Piperidin-1-yl-ethyl)-piperidine-1-carboxylic acid tert-butyl ester*

To 2.5 mL of neat piperidine (26 mmol) a solution of 4.9 g of 4-[2-(toluene-4-sulfonyloxy)-ethyl]-piperidine-1-carboxylic acid tert-butyl ester in 20 mL of $CH_3CN$ was added and the reaction was heated at 80˚C for about 6 hours. When TLC showed complete conversion the mixture was cooled down to room temperature and washed with 20 mL of saturated NaCl water solution. The organic phase was dried over $Na_2SO_4$ and then concentrated under reduced pressure. The oil obtained was purified by SiO2 column eluting with gradient starting from 100% DCM to DCM-NH3(2N MeOH solution) 9-1, affording 1.8 g of pure product (yield 46%).

Molecular formula: C17H32N2O2.

$^1$H-NMR (400MHz, CDC13): 1.08-1.14 (m,2H), 1.38-1.46 (m, 15H), 1.52-1.67 (m, 7H), 2.26-2.42 (m, 6H), 2.61-2.74 (m, 2H), 3.91-4.15 (m,2H).

*b) 4-(2-Piperidin-1-yl-ethyl)-piperidine*

To 44 mL of a solution of 6N HCl 4-(2-Piperidin-1-yl-ethyl)-piperidine-1-carboxylic acid tert-butyl ester was added and the reaction was stirred at room temperature for 10 minutes. The mixture was basified to pH 12 and the product was extracted with 20 mL of DCM. The organic phase was dried over $Na_2SO_4$ and then concentrated under reduced pressure affording 440 mg of the pure product (yield 37%).

Molecular formula: C12H24N2.

Mass (calculated) [196.34]; (found) $[M+H^+]$=197.

Lc Rt (10 min method)= 0.42, 100%.

*c) 4-(2-Piperidin-1-yl-ethyl)-piperidine-1-carboxylic acid (4-bromophenyl)-amide*

To a cooled solution of 4-(2-Piperidin-1-yl-ethyl)-piperidine (440 mg, 2.2 mmol) in dichloromethane (10 mL) p-bromophenylisocyanate (442 mg, 2.2 mmol) was added and the mixture stirred at 0˚C for 2 hours. The mixture was concentrated under reduced pressure and the residue was washed with $Et_2O$. The solid obtained was filtered giving 750 mg of pure product (yield 85%).

Molecular formula: C19H28BrN3O.

Mass (calculated) [394.36]; (found) $[M+H^+]$=394-396.

Lc Rt (10 min method)= 2.67, 92%.

*d) 4-(2-Piperidin-1-yl-ethyl)-piperidine-1-carboxylic acid (2'-fluoro-biphenyl-4-yl)-amide*

To a degassed solution of 4-(2-Piperidin-1-yl-ethyl)-piperidine-1-carboxylic acid (4-bromo-phenyl)-amide (100 mg, 0.25 mmol) in DME/$H_2O$ (1.8 ml/0.3 ml) 2-fluorophenyl boronic acid (55 mg, 0.39 mmol), $Na_2CO_3$ (55 mg, 0.52 mmol), $Pd(OAc)_2$ (6 mg, 10% mol) and tri-o-tolylphosphine (34 mg, 20% mol), were added. The solution was irradiated under microwave conditions for 20 minutes with power 200W.

The organic phase was diluted with 1 mL of AcOEt and separated.

The organic phase was loaded on SCX column and eluted with 10 mL of MeOH to remove triphenylphosphine oxide and then with NH3 (2N MeOH solution) to recover the pure product (25 mg, 25% yield).

Molecular formula: C25H32FN3O.

Mass (calculated) [409.55]; (found) $[M+H^+]$=410.

Lc Rt (10 min method)= 3.01, 100%.

$^1$H-NMR (400MHz, CDC13): 0.97-1.10 (m,2H), 1.27-1.32 (m, 3H), 1.43-1.49 (m, 4H), 1.58-1.70 (m, 2H), 2.18-2.37 (m, 4H), 2.67-2.78 (m, 2H), 4.03-4.11 (m,2H), 7.19-7.28 (m,2H), 7.29-7.36 (m,1H),7.36-7.41 (m, 2H), 7.43-7.49 9m, 1H), 7.51-7.56 (m, 2H), 8.55 (s,1H).

*Table 1- Examples 6-20*

Table 1 shows a selection of the compounds synthesised, which were prepared according to the method indicated in the last column of the table and discussed in detail in the Experimental Procedures with the synthesis of *Examples 1-5.* When the compound is indicated as the HCl salt, the salt was formed by dissolution of the free base in methanol and addition of 1 eq 1M HCl in ether followed by evaporation of the solvents. When the compound is indicated

as HCOOH (formic acid) salt, the compound was purified by preparative HPLC.

| Example | Structure | Salt | Parent Formula | Parent MW | Mass found | LC purity % | LC Rt | LC method (min) | Synthetic Method |
|---|---|---|---|---|---|---|---|---|---|
| 6 | 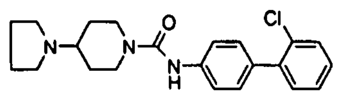 | | C22H26N3OCl | 383.91 | 385 | 100 | 2.42 | 10 | General procedure for cross-coupling reaction - thermal conditions |
| 7 | 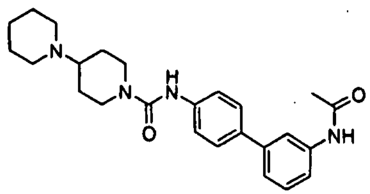 | | C25H32N4O2 | 420.55 | 421 | 100 | 2.11 | 10 | General procedure for cross-coupling reaction - microwave conditions - Tri-o-tolylphosphi ne |
| 8 | 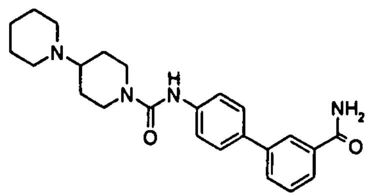 | | C24H30N4O2 | 406.52 | 407 | 100 | 2.01 | 10 | General procedure for cross-coupling reaction - microwave conditions - Tri-o-tolylphosphi ne |
| 9 | 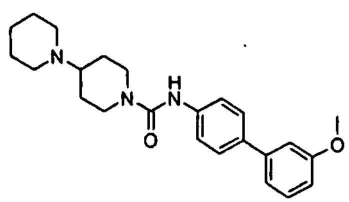 | | C24H31N3O2 | 393.52 | 394 | 98 | 2.53 | 10 | General procedure for cross-coupling reaction - microwave conditions -Tri-o-tolylphosphine |

(continued)

| Example | Structure | Salt | Parent Formula | Parent MW | Mass found | LC purity % | LC Rt | LC method (min) | Synthetic Method |
|---|---|---|---|---|---|---|---|---|---|
| 10 | 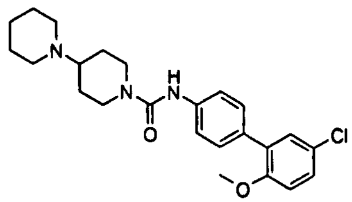 | | C24H30N3O2Cl | 427.97 | 428 | 100 | 2.83 | 10 | General procedure for cross-coupling reaction - microwave conditions - Tri-o-tolylphosphi ne |
| 11 | 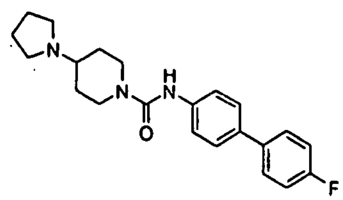 | | C22H26N3OF | 367.46 | 368 | 93 | 2.53 | 10 | General procedure for cross-coupling reaction - microwave conditions -Tri-o-tolylphosphine |
| 12 | 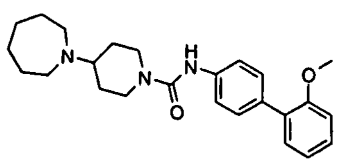 | | C25H33N3O2 | 407.55 | 408 | 97 | 2.56 | 10 | General procedure for cross-coupling reaction - microwave conditions -Tri-o-tolylphosphine |
| 13 | 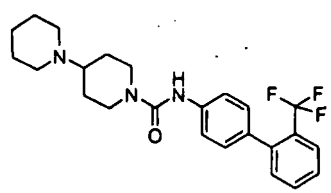 | HCOOH | C24H28N3OF3 | 431.49 | 432 | 100 | 2.91 | 10 | General procedure for cross-coupling reaction - microwave conditions -Tri-o-tolylphosphine |

(continued)

| Example | Structure | Salt | Parent Formula | Parent MW | Mass found | LC purity % | LC Rt | LC method (min) | Synthetic Method |
|---|---|---|---|---|---|---|---|---|---|
| 14 | 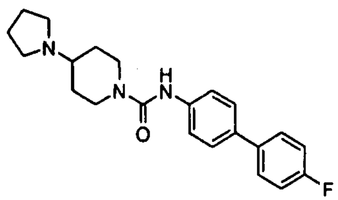 | | C22H26FN3O | 367 | 368 | 93 | 2.53 | 10 | General procedure for cross-coupling reaction - microwave conditions -Tri-o-tolylphosphine |
| 15 | 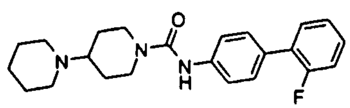 | HCOOH | C23H28FN3O | 381 | 382 | 99 | 2.51 | 10 | General procedure for cross-coupling reaction - microwave conditions -Tri-o-tolylphosphine |
| 16 | 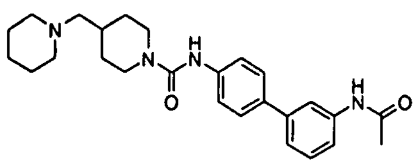 | | C26H34N4O2 | 434 | 435 | 100 | 2.29 | 10 | General procedure for cross-coupling reaction - microwave conditions -Tri-o-tolylphosphine |
| 17 | 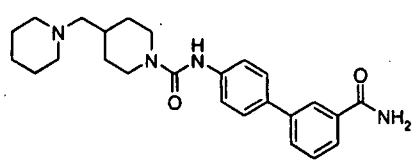 | | C25H32N4O2 | 420 | 421 | 100 | 2.13 | 10 | General procedure for cross-coupling reaction - microwave conditions -Tri-o-tolylphosphine |

(continued)

| Example | Structure | Salt | Parent Formula | Parent MW | Mass found | LC purity % | LC Rt | LC method (min) | Synthetic Method |
|---|---|---|---|---|---|---|---|---|---|
| 18 | 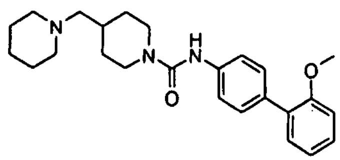 | HCOOH | C25H33N3O2 | 407 | 408 | 100 | 2.73 | 10 | General procedure for cross-coupling reaction - microwave conditions -Tri-o-tolylphosphine |
| 19 | 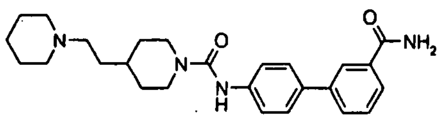 | | C26H34N4O2 | 434 | 435 | 100 | 2.24 | 10 | General procedure for cross-coupling reaction - microwave conditions -Tri-o-tolylphosphine |
| 20 | 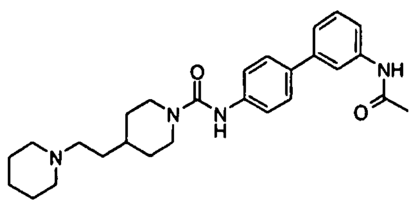 | HCOOH | C27H36N402 | 448 | 316 | 96 | 2.38 | 10 | General procedure for cross-coupling reaction - microwave conditions -Tri-o-tolylphosphine |

## Biological activity

*Cloning of alpha7 nicotinic acetylcholine receptor and generation of stable recombinant alpha7 nAChR expressing cell lines*

Full length cDNAs encoding the alpha7 nicotinic acetylcholine receptor were cloned from a rat brain cDNA library using standard molecular biology techniques. Rat GH4C1 cells were then transfected with the rat receptor, cloned and analyzed for functional alpha7 nicotinic receptor expression employing a FLIPR assay to measure changes in intracellular calcium concentrations. Cell clones showing the highest calcium-mediated fluorescence signals upon agonist (nicotine) application were further subcloned and subsequently stained with Texas red-labelled α-bungarotoxin (BgTX) to analyse the level and homogeneity of alpha7 nicotinic acetylcholine receptor expression using confocal microscopy. Three cell lines were then expanded and one characterised pharmacologically (see *Table 2* below) prior to its subsequent use for compound screening.

*Table 2 - Pharmacological characterisation of alpha7 nAChR stably expressed in GH4Cl cells using the functional FLIPR assay*

| Compound | $EC_{50}$ [microM] |
|---|---|
| Acetylcholine | 3.05 ± 0.08 (n=4) |
| Choline | 24.22 ± 8.30 (n=2) |
| Cytisine | 1.21 ± 0.13 (n=5) |
| DMPP | 0.98 ± 0.47 (n=6) |
| Epibatidine | 0.0 12± 0.002 (n=7) |
| Nicotine | 1.03 ± 0.26 (n=22) |

*Development of a functional FLIPR assay for primary screening*

A robust functional FLIPR assay (Z' = 0.68) employing the stable recombinant GH4Cl cell line was developed to screen the alpha7 nicotinic acetylcholine receptor. The FLIPR system allows the measurements of real time $Ca^{2+}$-concentration changes in living cells using a $Ca^{2+}$ sensitive fluorescence dye (such as Fluo4). This instrument enables the screening for agonists and antagonists for alpha 7 nAChR channels stably expressed in GH4Cl cells.

*Cell culture*

GH4Cl cells stably transfected with rat- alpha7-nAChR (see above) were used. These cells are poorly adherent and therefore pretreatment of flasks and plates with poly-D-lysine was carried out. Cells are grown in 150 $cm^2$ T-flasks, filled with 30 ml of medium at 37˚C and 5% $CO_2$.

*Data analysis*

$EC_{50}$ and $IC_{50}$ values were calculated using the IDBS *XLfit4.1* software package employing a sigmoidal concentration-response (variable slope) equation:

$$Y = Bottom + ((Top-Bottom)/(1+((EC_{50}/X)\ \hat{}\ HillSlope)).$$

*Assay validation*

The functional FLIPR assay was validated with the alpha7 nAChR agonists nicotine, cytisine, DMPP, epibatidine, choline and acetylcholine. Concentration-response curves were obtained in the concentration range from 0.001 to 30 microM. The resulting $EC_{50}$ values are listed in *Table* 2 and the obtained rank order of agonists is in agreement with published data (Quik et al., 1997)(22).

The assay was further validated with the specific alpha7 nAChR antagonist MLA (methyllycaconitine), which was used in the concentration range between 1 microM to 0.01 nM, together with a competing nicotine concentration

of 10 microM. The $IC_{50}$ value was calculated as 1.31±0.43 nM in nine independent experiments.

*Development of functional FLIPR assays for selectivity testing*

Functional FLIPR assays were developed in order to test the selectivity of compounds against the alpha1 (muscular) and alpha3 (ganglionic) nACh receptors and the structurally related 5-HT3 receptor. For determination of activity at alpha 1 receptors naively expressed in the rhabdomyosarcoma derived TE 671 cell line an assay employing membrane potential sensitive dyes was used, whereas alpha3 selectivity was determined by a calcium-monitoring assays using the native SH-SY5Y cell line. In order to test selectivity against the 5-HT3 receptor, a recombinant cell line was constructed expressing the human 5-HT3A receptor in HEK 293 cells and a calcium-monitoring FLIPR assay employed.

*Screening of compounds*

The compounds were tested using the functional FLIPR primary screening assay employing the stable recombinant GH4C1 cell line expressing the alpha7 nAChR. Hits identified were validated further by generation of concentration-response curves. The potency of compounds from *Examples 1-20* as measured in the functional FLIPR screening assay was found to range between 10 nM and 30 microM, with the majority showing a potency ranging between 10 nM and 10 microM.

**REFERENCES**

1. Prendergast, M. A., Harris, B. R., Mayer, S., Holley, R. C., Pauly, J. R., Littleton, J. M. (2001) Nicotine exposure reduces N-methyl-D-aspartate toxicity in the hippocampus: relation to distribution of the alpha7 nicotinic acetylcholine receptor subunit. Med.Sci.Monit. 7, 1153-1160.
2. Garrido, R., Mattson, M. P., Hennig, B., Toborek, M. (2001) Nicotine protects against arachidonic-acid-induced caspase activation, cytochrome c release and apoptosis of cultured spinal cord neurons. J.Neurochem. 76, 1395-1403.
3. Semba, J., Miyoshi, R., Kito, S. (1996) Nicotine protects against the dexamethasone potentiation of kainic acid-induced neurotoxicity in cultured hippocampal neurons. Brain Res. 735, 335-338.
4. Shimohama, S., Akaike, A., Kimura, J. (1996) Nicotine-induced protection against glutamate cytotoxicity. Nicotinic cholinergic receptor-mediated inhibition of nitric oxide formation. Ann.N.Y.Acad.Sci. 777, 356-361.
5. Akaike, A., Tamura, Y., Yokota, T., Shimohama, S., Kimura, J. (1994) Nicotine-induced protection of cultured cortical neurons against N- methyl-D-aspartate receptor-mediated glutamate cytotoxicity. Brain Res. 644, 181-187.
6. Yamashita, H., Nakamura, S. (1996) Nicotine rescues PC12 cells from death induced by nerve growth factor deprivation. Neurosci.Lett. 213, 145-147.
7. Shimohama, S., Greenwald, D. L., Shafron, D. H., Akaika, A., Maeda, T., Kaneko, S., Kimura, J., Simpkins, C. E., Day, A. L., Meyer, E. M. (1998) Nicotinic alpha 7 receptors protect against glutamate neurotoxicity and neuronal ischemic damage. Brain Res. 779, 359-363.
8. Socci, D. J., Arendash, G. W. (1996) Chronic nicotine treatment prevents neuronal loss in neocortex resulting from nucleus basalis lesions in young adult and aged rats. Mol. Chem.Neuropathol. 27, 285-305.
9. Rusted, J. M., Newhouse, P. A., Levin, E. D. (2000) Nicotinic treatment for degenerative neuropsychiatric disorders such as Alzheimer's disease and Parkinson's disease. Behav.Brain Res. 113, 121-129.
10. Kihara, T., Shimohama, S., Sawada, H., Kimura, J., Kume, T., Kochiyama, H., Maeda, T., Akaike, A. (1997) Nicotinic receptor stimulation protects neurons against beta-amyloid toxicity. Ann.Neurol. 42, 159-163
11. Kihara, T., Shimohama, S., Sawada, H., Honda, K., Nakamizo, T., Shibasaki, H., Kume, T., Akaike, A. (2001) alpha 7 nicotinic receptor transduces signals to phosphatidylinositol 3- kinase to block A beta-amyloid-induced neurotoxicity. J.Biol.Chem. 276, 13541-13546.
12. Kelton, M. C., Kahn, H. J., Conrath, C. L., Newhouse, P. A. (2000) The effects of nicotine on Parkinson's disease. Brain Cogn 43, 274-282.
13. Kem, W. R. (2000) The brain alpha7 nicotinic receptor may be an important therapeutic target for the treatment of Alzheimer's disease: studies with DMXBA (GTS-21). Behav.Brain Res. 113, 169-181.
14. Dajas-Bailador, F. A., Lima, P. A., Wonnacott, S. (2000) The alpha7 nicotinic acetylcholine receptor subtype mediates nicotine protection against NMDA excitotoxicity in primary hippocampal cultures through a Ca(2+) dependent mechanism. Neuropharmacology 39, 2799-2807.
15. Strahlendorf, J. C., Acosta, S., Miles, R., Strahlendorf, H. K. (2001) Choline blocks AMPA-induced dark cell

degeneration of Purkinje neurons: potential role of the alpha7 nicotinic receptor. Brain Res. 901, 71-78.
16. Jonnala, R. R., Terry, A. V., Jr., Buccafusco, J. J. (2002) Nicotine increases the expression of high affinity nerve growth factor receptors in both in vitro and in vivo. Life Sci. 70, 1543-1554.
17. Bencherif, M., Bane, A. J., Miller, C. H., Dull, G. M., Gatto, G. J. (2000) TC-2559: a novel orally active ligand selective at neuronal acetylcholine receptors. Eur.J.Pharmacol. 409, 45-55. Ref Type: Journal
18. Donnelly-Roberts, D. L., Xue, I. C., Arneric, S. P., Sullivan, J. P. (1996) In vitro neuroprotective properties of the novel cholinergic channel activator (ChCA), ABT-418. Brain Res. 719, 36-44.
19. Meyer, E. M., Tay, E. T., Zoltewicz, J. A., Meyers, C., King, M. A., Papke, R. L., De Fiebre, C. M. (1998) Neuroprotective and memory-related actions of novel alpha-7 nicotinic agents with different mixed agonist/antagonist properties. J.Pharmacol.Exp.Ther. 284, 1026-1032.
20. Stevens, T. R., Krueger, S. K., Fizsimonds, R. M. and Picciotto, M. R. (2003) Neuroprotection by nicotine in mouse primary cortical cultures involves activation of calcineurin and L-type calcium channel inactivation. J. Neuroscience 23, 10093-10099.
21. Wang H, Yu M, Ochani M, Amella CA, Tanovic M, Susarla S, Li JH, Wang H, Yang H, Ulloa L, Al-Abed Y, Czura CJ, Tracey KJ (2003) Nicotinic acetylcholine receptor alpha7 subunit is an essential regulator of inflammation. Nature 421:384-388.
22. Quik M, Philie J. and Choremis J. (1997). Modulation of alpha7 nicotinic receptor-mediated calcium influx by nicotinic agonists. Mol. Pharmacol., 51, 499-506.

## Claims

**1.** A compound of formula I

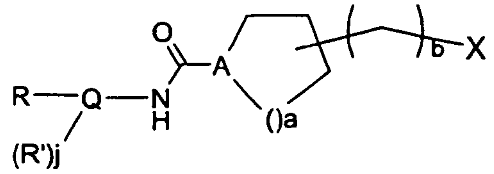

(I)

wherein:

A is N;
a is an integer from 1 to 3;
bis 0, 1, or 2;
X is a group of formula:

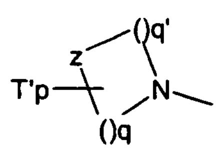

wherein:

Z is $CH_2$, N, O;
p is 0 or 1;
T' represent, independently from one another when p is greater than 1, linear, branched or cyclic ($C_1$-$C_6$) alkyl,

trihaloalkyl, hydroxyalkyl, alkoxy, alkylcarbonyl, alkoxycarbonyl, alkylcarbonylamino; linear, branched or cyclic ($C_1$-$C_6$) alkoxy-($C_1$-$C_6$) alkyl; linear, branched or cyclic ($C_1$-$C_6$) alkylaminocarbonyl; carbamoyl; or, when p is 2 or 3, two T' substituents, with the atoms of the X ring they are attached to, form a 5- to 8-membered ring with spiro or fused junction;
q and q' are, independently from one another, integers from 1 to 4;
Q is a 5 to 10-membered aromatic or heteroaromatic ring;
R represents a 5 to 10-membered aromatic or heteroaromatic ring optionally substituted with one or more groups selected from: halogen; hydroxy; mercapto; cyano; nitro; amino; linear, branched or cyclic ($C_1$-$C_6$) alkyl, trihaloalkyl, alkoxy or alkylcarbonyl; linear, branched, or cyclic ($C_1$-$C_6$) alkylcarbonylamino; mono- or di, linear, branched, or cyclic ($C_1$-$C_6$) alkylamino or alkylaminocarbonyl; carbamoyl; linear, branched or cyclic ($C_1$-$C_6$) alkoxy-($C_1$-$C_6$) alkyl;
j is 0, 1 or 2;
R' represent, independently of one another when j = 2, halogen; hydroxy; trihalomethyl; trihalomethoxy; linear, branched or cyclic ($C_1$-$C_6$) alkyl, trihaloalkyl, alkoxy, hydroxyalkyl.

**2.** A compound according to claim 1, wherein:

AisN;
a is an integer from 1 to 2;
b is 0, 1 or 2;
X is a group of formula:

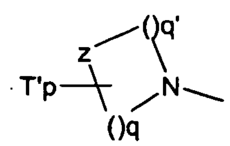

wherein:

Z is $CH_2$;
p is 0;
q and q' are, independently from one another, integers from 1 to 3;
Q is a 5 to 10-membered aromatic ring;
R represents a 5 to 10-membered aromatic or heteroaromatic ring optionally substituted with one or more groups selected from: halogen; linear, branched or cyclic ($C_1$-$C_3$) alkyl, alkoxy; linear, branched, or cyclic ($C_1$-$C_3$) alkylcarbonylamino; mono- or di, linear, branched, or cyclic ($C_1$-$C_3$) alkylaminocarbonyl; carbamoyl;
j is 0.

**3.** A compound according to claim 2, wherein both Q and R are phenyl.

**4.** A pharmaceutical composition containing a compound according to claims 1-3 and a pharmaceutically acceptable carrier or excipient.

**5.** The use of a compound according to claims 1-3, for the preparation of a medicament for the treatment of neurological, psychiatric, cognitive, immunological and inflammatory disorders.

**6.** The use according to claim 5, for the treatment of neurdegenerative diseases.

**7.** The use according to claims 5 and 6, for the treatment of senile dementia, attention deficit disorders, Alzheimer's disease and schizophrenia.

## Patentansprüche

**1.** Eine Verbindung der Formel I

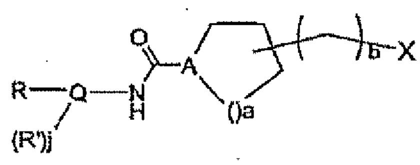

(I)

wobei:

A N ist;
a eine ganze Zahl von 1 bis 3 ist;
b 0, 1 oder 2 ist;
X eine Gruppe der folgenden Formel ist:

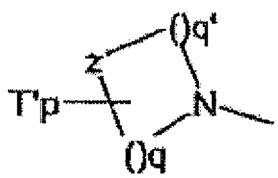

wobei:

Z $CH_2$, N, O ist;
p 0 oder 1 ist;
T', unabhängig von einander, wenn p größer als 1 ist, lineare, verzweigte oder zyklische ($C_1$-$C_6$) Alkyl-, Trihaloalkyl-, Hydroxyalkyl-, Alkoxy-, Alkylcarbonyl-, Alkoxycarbonyl-, Alkylcarbonylaminogruppen; lineare, verzweigte oder zyklische ($C_1$-$C_6$) Alkoxy-($C_1$-$C_6$) Alkyle; lineare, verzweigte oder zyklische ($C_1$-$C_6$) Alkylaminocarbonylgruppen; Carbamoyle darstellt; oder, wenn p 2 oder 3 ist, zwei T' Substituenten mit den Atomen des X-Rings, an den sie gebunden sind, ein fünf- bis achtgliedriges Ringsystem mit Verknüpfung oder kondensierter Verknüpfung bilden;
q und q', unabhängig von einander, ganze Zahlen von 1 bis 4 sind;
Q ein fünf- bis zehngliedrigen aromatisches oder heteroaromatisches Ringsystem ist;
R ein fünf- bis zehngliedriges aromatisches oder heteroaromatisches Ringsystem ist, das optional substituiert ist mit einer oder mehreren Gruppen ausgewählt aus: Halogen-; Hydroxy-; Mercapto-; Zyano-; Nitro-; Aminogruppen; linearen, verzweigten oder zyklischen ($C_1$-$C_6$) Alkyl-, Trihaloalkyl-, Alkoxy- oder Alkylcarbonylgruppen; linearen, verzweigten oder zyklischen ($C_1$-$C_6$) Alkylcarbonylaminogruppen; Mono- oder Di-, linearen, verzweigten oder zyklischen ($C_1$-$C_6$) Alkylamino- oder Alkylaminocarbonylgruppen; Carbamoylgruppen; linearen, verzweigten oder zyklischen ($C_1$-$C_6$) Alkoxy-($C_1$-$C_6$) Alkylgruppen;
j 0, 1 oder 2 ist;
R', unabhängig von einander wenn j = 2, Halogen-; Hydroxy-; Trihalomethyl-; Trihalomethoxygruppen; lineare, verzweigte oder zyklische ($C_1$-$C_6$) Alkyl- Trihaloalkyl-Alkoxy-, Hydroxialkylgruppen darstellt.

**2.** Eine Verbindung gemäß Anspruch 1, wobei:

A N ist;
a eine ganze Zahl von 1 bis 2 ist;
b 0, 1 oder 2 ist;
X eine Gruppe der folgenden Formel ist:

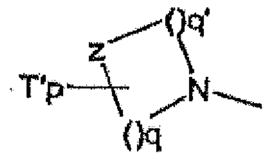

wobei:

Z $CH_2$ ist;
p 0 ist;
q und q', unabhängig von einander, ganze Zahlen von 1 bis 3 sind;
Q ein fünf- bis zehngliedriges aromatisches Ringsystem ist;
R ein fünf- bis zehngliedriges aromatisches oder heteroaromatisches Ringsystem darstellt, das optional substituiert ist mit einer oder mehreren Gruppen ausgewählt aus: Halogen-; linearen, verzweigten oder zyklischen ($C_1$-$C_3$) Alkyl-, Alkoxy-; linearen, verzweigten oder zyklischen ($C_1$-$C_3$) Alkylcarbonylamino-; Mono- oder Di-, linearen, verzweigten, oder zyklischen ($C_1$-$C_3$) Alkyllaminocarbonyl-; Carbamoylgruppen;
j 0 ist.

3. Eine Verbindung gemäß Anspruch 2, wobei Q und R beide Phenylgruppen sind.

4. Eine pharmazeutische Zusammensetzung enthaltend eine Verbindung gemäß der Ansprüche 1-3 und einen pharmazeurisch verträglichen Trägerstoff oder Hilfsstoff.

5. Verwendung einer Verbindung gemäß der Ansprüche 1-3 für die Herstellung eines Medikamentes zur Behandlung von neurologischen, psychiatrischen, kognitiven, immunologischen und entzündliche Erkrankungen.

6. Verwendung gemäß Anspruch 5 für die Behandlung von neurodegenerativen Erkrankungen.

7. Verwendung gemäß der Ansprüche 5 und 6 für die Behandlung von Altersdemenz, Aufmerksamkeitsdefizitstörungen, Morbus Alzheimer und Schizophrenie.

## Revendications

1. Composé de formule I

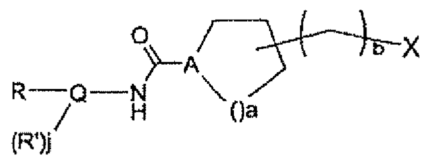

(I)

dans laquelle :

A est N;
a est un nombre entier de 1 à 3 ;
b est 0, 1, ou 2 ;
X est un groupe de formule :

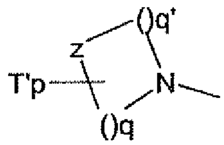

où :

Z est $CH_2$, N, O ;
p est 0 ou 1 ;
T' représentent, indépendamment les uns des autres quand p est plus grand que 1, ($C_1$-$C_6$) alkyle, trihaloalkyle, hydroxyalkyle, alkoxy, alkylcarbonyle, alkoxycarbonyle, alkylcarbonylamino, linéaire, ramifié ou cyclique; ($C_1$-$C_6$) alkoxy ($C_1$-$C_6$) alkyle linéaire, ramifié ou cyclique; ($C_1$-$C_6$) alkylaminocarbonyle linéaire, ramifié ou cyclique ; carbamoyle ; ou, quand p est 2 ou 3, deux substituants T', avec les atomes du cycle X auquel ils sont attachés, forment un cycle de 5 à 8 membres avec la jonction spiro ou fusionnée;
q et q' sont, indépendamment les uns des autres, les nombres entiers de 1 à 4 ;
Q est un cycle aromatique ou hétéroaromatique de 5 à 10 membres ;
R représente un cycle aromatique ou hétéroaromatique de 5 à 10 membres optionnellement substitué avec un ou plusieurs groupes sélectionnés parmi: halogène ; hydroxy ; mercapto ; cyano ; nitro ; amino ; ($C_1$-$C_6$) alkyle, trihaloalkyle, alkoxy ou alkylcarbonyle, linéaire, ramifié ou cyclique; ($C_1$-$C_6$) alkylcarbonylamino linéaire, ramifié, ou cyclique ; mono- ou di- ($C_1$-$C_6$) alkylamino ou alkylaminocarbonyle, linéaire, ramifié ou cyclique; carbamoyl ; ($C_1$-$C_6$) alkoxy ($C_1$-$C_6$) alkyle linéaire, ramifié ou cyclique;
j est 0, 1 ou 2 ;
R' représentent, indépendamment les uns des autres quand j = 2, halogène ; hydroxy ; trihalométhyle ; trihalométhoxy ; ($C_1$-$C_6$) alkyle, trihaloalkyle, alkoxy, hydroxyalkyle, linéaire, ramifié ou cyclique.

**2.** Composé selon la revendication 1, où :

A est N ;
a est un nombre entier de 1 à 2 ;
b est 0, 1, ou 2 ;
X est un groupe de formule :

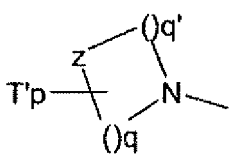

où :

Z est $CH_2$ ;
p est 0 ;
q et q' sont, indépendamment les uns des autres, les nombres entiers de 1 à 3 ;
Q est un cycle aromatique de 5 à 10 membres ;
R représente un cycle aromatique ou hétéroaromatique de 5 à 10 membres optionnellement substitué avec un ou plusieurs groupes sélectionnés parmi : halogène, ($C_1$-$C_3$) alkyle, alkoxy, linéaire, ramifié ou cyclique; ($C_1$-$C_3$) alkylcarbonylamino linéaire, ramifié, ou cyclique ; mono- ou di- ($C_1$-$C_3$) alkylaminocarbonyle linéaire, ramifié ou cyclique ; carbamoyle ;
j est 0.

**3.** Composé selon la revendication 2, où Q et R sont tous les deux un phényle.

4. Composition pharmaceutique contenant un composé selon les revendications 1 à 3 et un porteur ou excipient pharmaceutiquement acceptable.

5. Utilisation d'un composé selon des revendications 1 à 3, pour la préparation d'un médicament pour le traitement des désordres neurologiques, psychiatriques, cognitifs, immunologiques et inflammatoires.

6. Utilisation selon la revendication 5, pour le traitement de maladies neurodégénératives.

7. Utilisation selon les revendications 5 et 6, pour le traitement de la démence sénile, des désordres de déficit de l'attention, de la maladie d'Alzheimer et de la schizophrénie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 652852 A
- WO 2001005763 A
- WO 9950247 A
- WO 2004056799 A
- WO 2004056805 A
- WO 02072570 A
- WO 0130348 A
- WO 200411045 A
- WO 2004110415 A


### Non-patent literature cited in the description


- **Greene, T. ; Wuts, P. G. M.** Protective Groups in Organic Synthesis. John Wiley and Sons, 1999
- **Suzuki, A.** *Pure and Appl. Chem.,* 1994, vol. 66, 213-222
- Remington's Pharmaceutical Science. Mack Publishing Company
- **Still, C.** *J. Org Chem,* 1978, vol. 43, 2923
- **Prendergast, M. A. ; Harris, B. R. ; Mayer, S. ; Holley, R. C. ; Pauly, J. R. ; Littleton, J. M.** Nicotine exposure reduces N-methyl-D-aspartate toxicity in the hippocampus: relation to distribution of the alpha7 nicotinic acetylcholine receptor subunit. *Med.Sci.Monit.,* 2001, vol. 7, 1153-1160
- **Garrido, R. ; Mattson, M. P. ; Hennig, B. ; Toborek, M.** Nicotine protects against arachidonic-acid-induced caspase activation, cytochrome c release and apoptosis of cultured spinal cord neurons. *J.Neurochem.,* 2001, vol. 76, 1395-1403
- **Semba, J. ; Miyoshi, R. ; Kito, S.** Nicotine protects against the dexamethasone potentiation of kainic acid- induced neurotoxicity in cultured hippocampal neurons. *Brain Res.,* 1996, vol. 735, 335-338
- **Shimohama, S. ; Akaike, A. ; Kimura, J.** Nicotine-induced protection against glutamate cytotoxicity. Nicotinic cholinergic receptor-mediated inhibition of nitric oxide formation. *Ann.N.Y.Acad.Sci.,* 1996, vol. 777, 356-361
- **Akaike, A. ; Tamura, Y. ; Yokota, T. ; Shimohama, S. ; Kimura, J.** Nicotine-induced protection of cultured cortical neurons against N- methyl-D-aspartate receptor-mediated glutamate cytotoxicity. *Brain Res.,* 1994, vol. 644, 181-187
- **Yamashita, H. ; Nakamura, S.** Nicotine rescues PC12 cells from death induced by nerve growth factor deprivation. *Neurosci.Lett.,* 1996, vol. 213, 145-147
- **Shimohama, S. ; Greenwald, D. L. ; Shafron, D. H. ; Akaika, A. ; Maeda, T. ; Kaneko, S. ; Kimura, J. ; Simpkins, C. E. ; Day, A. L. ; Meyer, E. M.** Nicotinic alpha 7 receptors protect against glutamate neurotoxicity and neuronal ischemic damage. *Brain Res.,* 1998, vol. 779, 359-363
- **Socci, D. J. ; Arendash, G. W.** Chronic nicotine treatment prevents neuronal loss in neocortex resulting from nucleus basalis lesions in young adult and aged rats. *Mol. Chem.Neuropathol.,* 1996, vol. 27, 285-305
- **Rusted, J. M. ; Newhouse, P. A. ; Levin, E. D.** Nicotinic treatment for degenerative neuropsychiatric disorders such as Alzheimer's disease and Parkinson's disease. *Behav.Brain Res.,* 2000, vol. 113, 121-129
- **Kihara, T. ; Shimohama, S. ; Sawada, H. ; Kimura, J. ; Kume, T. ; Kochiyama, H. ; Maeda, T. ; Akaike, A.** Nicotinic receptor stimulation protects neurons against beta-amyloid toxicity. *Ann.Neurol.,* 1997, vol. 42, 159-163
- **Kihara, T. ; Shimohama, S. ; Sawada, H. ; Honda, K. ; Nakamizo, T. ; Shibasaki, H. ; Kume, T. ; Akaike, A.** alpha 7 nicotinic receptor transduces signals to phosphatidylinositol 3- kinase to block A beta-amyloid-induced neurotoxicity. *J.Biol.Chem.,* 2001, vol. 276, 13541-13546
- **Kelton, M. C. ; Kahn, H. J. ; Conrath, C. L. ; Newhouse, P. A.** The effects of nicotine on Parkinson's disease. *Brain Cogn,* 2000, vol. 43, 274-282
- **Kem, W. R.** The brain alpha7 nicotinic receptor may be an important therapeutic target for the treatment of Alzheimer's disease: studies with DMXBA (GTS-21). *Behav.Brain Res.,* 2000, vol. 113, 169-181

- **Dajas-Bailador, F. A. ; Lima, P. A. ; Wonnacott, S.** The alpha7 nicotinic acetylcholine receptor subtype mediates nicotine protection against NMDA excitotoxicity in primary hippocampal cultures through a Ca(2+) dependent mechanism. *Neuropharmacology,* 2000, vol. 39, 2799-2807
- **Strahlendorf, J. C. ; Acosta, S. ; Miles, R. ; Strahlendorf, H. K.** Choline blocks AMPA-induced dark cell degeneration of Purkinje neurons: potential role of the alpha7 nicotinic receptor. *Brain Res.,* 2001, vol. 901, 71-78
- **Jonnala, R. R. ; Terry, A. V., Jr. ; Buccafusco, J. J.** Nicotine increases the expression of high affinity nerve growth factor receptors in both in vitro and in vivo. *Life Sci.,* 2002, vol. 70, 1543-1554
- **Bencherif, M. ; Bane, A. J. ; Miller, C. H. ; Dull, G. M. ; Gatto, G. J.** TC-2559: a novel orally active ligand selective at neuronal acetylcholine receptors. *Eur.J.Pharmacol.,* 2000, vol. 409, 45-55
- **Donnelly-Roberts, D. L. ; Xue, I. C. ; Arneric, S. P. ; Sullivan, J. P.** In vitro neuroprotective properties of the novel cholinergic channel activator (ChCA), ABT-418. *Brain Res.,* 1996, vol. 719, 36-44
- **Meyer, E. M. ; Tay, E. T. ; Zoltewicz, J. A. ; Meyers, C. ; King, M. A. ; Papke, R. L. ; De Fiebre, C. M.** Neuroprotective and memory-related actions of novel alpha-7 nicotinic agents with different mixed agonist/antagonist properties. *J.Pharmacol.Exp.Ther.,* 1998, vol. 284, 1026-1032
- **Stevens, T. R. ; Krueger, S. K. ; Fizsimonds, R. M. ; Picciotto, M. R.** Neuroprotection by nicotine in mouse primary cortical cultures involves activation of calcineurin and L-type calcium channel inactivation. *J. Neuroscience,* 2003, vol. 23, 10093-10099
- **Wang H ; Yu M ; Ochani M ; Amella CA ; Tanovic M ; Susarla S ; Li JH ; Wang H ; Yang H ; Ulloa L.** Nicotinic acetylcholine receptor alpha7 subunit is an essential regulator of inflammation. *Nature,* 2003, vol. 421, 384-388
- **Quik M ; Philie J. ; Choremis J.** Modulation of alpha7 nicotinic receptor-mediated calcium influx by nicotinic agonists. *Mol. Pharmacol.,* 1997, vol. 51, 499-506